# EUROPEAN PATENT APPLICATION

(11) **EP 4 005 557 A1**
(43) Date of publication of application: **01.06.2022**
(21) Application number: 20210470.9
(22) Date of filing: 27.11.2020
(51) Int. Cl.: A61K 9/127, A61K 9/51, A61K 31/00

(54) **METHOD FOR PREPARING NANOSYSTEMS**

(71) Applicant: Université de Genève, 1211 Genève 4 (CH)
(72) Inventor: Kalia, Yogeshvar N., 1232 CONFIGNON (CH); Darade, Aditya Ramnath, 1206 GENEVE (CH)
(74) Representative: Casalonga

(57) **Abstract**

The present invention deals with a method for preparing a solid premix comprising cryomilling a composition comprising one or more amphiphilic compounds and one or more water-soluble solutes.

The invention aims at a method for preparing a nanosystem vehicle, preferably a vesicular nanosystem, comprising cryomilling a composition comprising one or more amphiphilic compounds and one or more water-soluble solutes in order to obtain a solid premix and dispersing said solid premix into an aqueous medium.

## Description

### Field of the invention

The present invention deals with a method for preparing a nanosystem vehicle, preferably a vesicular nanosystem, comprising cryomilling a composition comprising one or more amphiphilic compounds and one or more water-soluble solutes in order to obtain a solid premix and dispersing said solid premix into an aqueous medium.

The invention further relates to a method for preparing a solid premix comprising cryomilling a composition comprising one or more amphiphilic compounds and one or more water-soluble solutes.

The invention also pertains to a solid premix obtainable by the aforementioned method and its uses for therapeutic and non-therapeutic applications.

### Background of the invention

Nanosystem vehicles, especially vesicular nanosystems, are currently often used in therapeutic applications, such as for encapsulating and delivering biological active ingredients, for example drugs, at a controlled rate directly to the targeted sites in the body, e.g. inflamed tissues and/or organs, in order to provide an efficient and adequate therapeutic action over the entire period of treatment, or for stabilizing said biological active ingredients, such as messenger RNA (mRNA) in gene therapy.

Nanosystem vehicles have also been developed and successfully used in non-therapeutic applications, for example in the preparation of topical formulations intended for cosmetic and/or dermatological purposes. Indeed, nanosystems can help penetrate or absorb cosmetic or dermatologic active ingredients within epidermal layers and/or in the dermis in a controlled manner to achieve burst and/or sustain release of said active ingredients.

Nanosystem vehicles can include monolayer vesicle structures, especially micelles such as polymeric micelles, as well as bilayer vesicle structures, such as liposomes, niosomes, sphingosomes, transferosomes, ethosomes and so forth. These vesicle nanosystems are generally formed by the self-association of amphiphilic compounds when they are added to an aqueous solution. In particular, the active ingredients which may be hydrophilic or hydrophobic can be added during or after the formation of said nanosystems vehicles. In case of active drug loading, the pharmaceutical active ingredient is added during the formation of said vehicles and in case of passive drug loading, the pharmaceutical active ingredient is added after such formation.

Currently available methods for preparing such nanosystem vehicles generally involve the implementation and subsequent removal of organic solvents.

For instance, liposomes are generally made with amphiphilic lipids, e.g. phospholipids that are first dissolved and then mixed in an organic solvent, such as a chloroform/methanol mixture, in order to obtain a homogenous mixture of lipids. After being thoroughly mixed, the organic solvent is then removed by evaporation, for example by using a dry nitrogen or argon stream or by rotary evaporation under reduced pressure, to yield a thin lipid film. The film is then hydrated with an aqueous or saline solution which may contain the active ingredient. The dispersion of phospholipids in the aqueous or saline medium leads to the formation of liposomes.

However, it bespeaks that when such methods are implemented for the bulk production of the aforementioned nanosystem vehicles, considerable amounts of organic solvents may be required and then later be discarded by additional steps which can be tedious to carry out. In particular, the complexity of implementing such additional steps may hinge on the nature of the organic solvents. As a consequence, the use and later removal of organic solvents will cause the costs to soar and lengthen the overall manufacturing process.

Furthermore, the use and subsequent removal of organic solvents can also trigger safety issues and health hazards to the operators in industrial production as they are combustible, extremely inflammable and their inhalation can cause a string of serious and various ailments. Besides, the organic solvents should be transported and stored under standardized conditions, which impose additional stringent conditions and ratchet up the industrial costs of the final formulations.

The removal of the organic solvents can also generate wastes that must be properly treated in order to mitigate potential adverse effects caused to the environment.

In addition, the operations of removing the organic solvents may turn out to be not efficient enough and lead to the presence of residues in nanosystem vehicles that can alter the toxicity and the properties of the obtained formulations.

Especially, the residual presence of organic solvents may impede the stability of the final formulations and/or hinder the incorporation, and therefore the action, of the active ingredients in the nanosystem vehicles.

More precisely, in some cases, the organic solvents may weaken the percentage of inclusion of active ingredients and hamper their activity, especially in case of biological active ingredients.

As a consequence, the manufacturing steps carried out to get rid of the organic solvents should be carefully and closely monitored to ensure the absence of residues in the final formulations.

Moreover, in case of active drug loadings, the additional steps that are carried out in an attempt to eliminate organic solvents may also degrade or remove at the same time parts of the active ingredients, which had been loaded. Therefore, drugs can be lost or degraded during conventional manufacturing processes.

In other words, the use and later removal of organic solvents can thus be challenging for bulk production of nanosystems media in terms of controlling the costs, ensuring safety and health issues, and obtaining non-toxic, stable and efficient final nanoformulations.

As a result, it remains a real need to provide a method for producing nanosystem vehicles which is easier to implement for bulk production than the current available methods which is also able to ensure safety conditions, both for the operator and the environment, and to lead to stable and efficient formulations.

Specifically, one of the goals of the present invention is to provide a method that is able to alleviate the several flaws occurring in the current available methods, in particular, the risks of solvent hazards, both for the operator and the environment, the number of complicated manufacturing steps required to remove organic solvents and the presence of impurities that may impede the toxicity and the properties of the obtained nanoformulations.

More specifically, another goal of the present invention is to avoid the use and later removal of organic solvents during the manufacture of a nanosystem vehicle.

### Description of the invention

The present invention namely results from the unexpected findings, by the inventors, that the implementation of cryomilling of a composition comprising one or more amphiphilic compounds and one or more water-soluble solutes is able to lead to a solid and ready to dissolve premix which forms afterwards a stable nanosystem medium upon contact with an aqueous medium, preferably water.

Indeed, the implementation of cryomilling is able to fraction and reduce the size of a composition comprising one or more amphiphilic compounds and one or more water-soluble solutes in a cooled environment in order to produce a solid mixture of said composition, called premix, which has the advantage of displaying a high surface area. In particular, the rheology and physical features of said solid mixture are altered as compared to those of the individual components present in the composition. When this solid mixture is dispersed in an aqueous medium, due to the high surface area and capillary effect of water-soluble solutes, the dissolution is increased causing the formation, in particular the immediate formation in some advantageous cases, of the nanosystem vehicles.

Hence, the method according to the present invention does not require the use and subsequent removal of organic solvents which makes it easier and cheaper to implement for bulk production on an industrial scale than the current available methods while ensuring safety and healthy conditions for the operator.

Especially, the method of the present invention displays the advantage of not depending on the use of an organic solvent which implies that the risks linked to solvent hazards, both for the operator and the environment, the number of manufacturing steps that could be complicated to carry out in order to remove organic solvents, and the possible residual impurities that may alter the toxicity and the properties of the obtained formulations are preferably avoided unlike the current available methods.

More precisely, the method of the present invention is also able to narrow down the unpredictable variables due to the presence of the organic solvents or linked to the operations designed to get rid of them.

Therefore, the present invention pertains to a method for preparing a solid premix comprising cryomilling, a composition comprising one or more amphiphilic compounds and one or more water-soluble solutes.

As previously mentioned, the dispersion in an aqueous medium, especially water, of the solid premix leads to the formation of stable nanosystem vehicles. In particular, nanosystem vehicles, preferably vesicular nanosystems such as micelles and liposomes, may instantaneously be formed upon contact with an aqueous medium and are ready to be used.

This method is advantageous as it leads to the formation of a solid premix which can be used afterwards for therapeutic or non-therapeutic applications.

Thus, the present invention also relates to a method for preparing a nanosystem vehicle comprising:
i) cryomilling a composition comprising one or more amphiphilic compounds and one or more water-soluble solutes in order to obtain a solid premix,
ii) dispersing the solid premix into an aqueous medium.

Indeed, the method according to the present invention is able to produce stable nanosystem vehicles, especially stable vesicular nanosystem, as the solid premix can enhance the shelf life of formulations along with easy packaging and transportation.

In addition, the method of the present invention is able to incorporate a higher percentage of active ingredients than current conventional methods without hampering their activity and stability.

In other words, the activity of the active ingredients is less likely to be degraded in the nanosystems obtained by the aforementioned method of the present invention than by conventional methods. Especially, the activity of biological active ingredients, preferably those that are sensitive to the presence of organic solvents, may be enhanced.

As a consequence, it bespeaks that a treatment based on the administration of such nanosystem vehicles may be more efficient as more active ingredients may be delivered to the targeted sites.

Moreover, the method for preparing a nanosystem vehicle according to the invention can be easily optimized and monitored.

Another aspect of the present invention relies on a solid premix obtainable from a method comprising cryomilling a composition comprising one or more amphiphilic compounds and one or more water-soluble solutes.

Once again, as previously mentioned, the solid premix displays the advantage of being easy to dissolve in an aqueous medium, especially water, in order to form sometimes almost instantaneously nanosystem vehicles.

The solid premix is also easy to package and transport which considerably eases its use.

On one hand, the solid premix may be used in therapeutic application, preferably in order to deliver drug substances in the body, especially drug substances to targeted sites in the body.

Hence, another subject-matter of the present invention concerns the solid premix as previously described for its use in drug delivery, preferably to targeted sites in the body, containing one or more biological active ingredients, preferably chosen from pharmaceutical active ingredients.

In the same manner, the invention is directed to a nanosystem vehicle, obtainable from the method previously defined, for its use in a therapeutic treatment and/or *in vivo* diagnosis method comprising one or more biological active ingredients, preferably chosen from pharmaceutical active ingredients.

On the other hand, the solid premix can also be used for non-therapeutic purposes as well.

Another subject-matter of the present invention concerns the use of a solid premix as previously described for a non-therapeutic application selected from the group consisting of loading active ingredients, stabilizing messenger RNA (mRNA) or preparing a topical non-therapeutic composition, preferably a cosmetic composition.

Other subjects and characteristics, aspects and advantages of the invention will emerge even more clearly on reading the description and the example that follows.

In the text herein below, and unless otherwise indicated, the limits of a range of values are included in that range, in particular in the expressions "between" and "ranging from ... to ...".

Moreover, the expression "at least one" used in the present description is equivalent to the expression "one or more".

The term "dispersion" encompasses the concept of suspension and emulsions.

### Method for preparing the premix

As intended herein, the term "comprising" has the meaning of "including" or "containing", which means that when an object "comprises" one or several elements, other elements than those mentioned may also be included in the object. In contrast, when an object is said to "consist of' one or several elements, the object is limited to the listed elements and cannot include other elements than those mentioned.

As previously detailed, the present invention is directed to a method for preparing a solid premix comprising cryomilling a composition comprising one or more amphiphilic compounds and one or more water-soluble solutes.

In particular, cryomilling allows to mill said composition in a cooled environment and relies on impact and attrition to reduce the size of said composition in order to obtain nanometric sized particles.

In accordance with the present invention, cryomilling can take place within a high energy mill, such as an attritor mill, for example an attritor mill having metallic or ceramic balls.

An example of high energy mill in order to perform cryomilling can be SPEX^{®} SamplePrep cryomill, Retsch^{®} cryomill, AIR PRODUCTS cryomill.

Preferably, cryomilling is performed at a cryogenics temperature. In other words, the composition is milled at a cryogenics temperature.

According to the present invention, the expression "cryogenics temperature" stands for a temperature that is equal to or lower than -150°C, especially lower than - 180°C.

Preferably, the composition is milled in a cryogenic fluid cooled environment.

According to the present invention, the expression "a cryogenic fluid cooled environment" means that the composition is placed in an environment that is cooled by a cryogenic fluid. In other words, cryomilling preferably occurs in an environment cooled by a cryogenic fluid.

In addition, the feature "cryogenic fluid" refers to a fluid whose boiling point at atmospheric pressure is equal to or lower than -150°C. In particular, cryogenic fluid refers to a liquified gas that is kept in its liquid state at a temperature equal or lower than -150°C, especially lower than -180°C.

Cryogenic fluid can be selected from the group consisting of liquid nitrogen or liquid argon, preferably liquid nitrogen.

Preferably, cryomilling is performed in a liquid nitrogen cooled environment. In other words, the composition is milled in a liquid nitrogen cooled environment, especially said composition is immersed in liquid nitrogen during milling.

Advantageously, cryomilling is performed in an attritor mill in a cryogenic fluid, especially in a liquid nitrogen, cooled environment.

Cryomilling can be carried out for a period of time ranging from 1 min to 360 minutes, preferably from 5 minutes to 60 minutes.

The amphiphilic compound can be selected from the group consisting of polymers, surfactants, lipids and mixtures thereof, especially lipids, surfactants, and mixtures thereof, more especially in the group consisting of lipids.

The amphiphilic compound may be an amphiphilic lipid selected from the group consisting of phospholipids, synthetic phospholipids, glycerolipids, sphingolipids, glycosphingolipids and mixtures thereof.

The amphiphilic compound is preferably an amphiphilic lipid selected from the group consisting of phospholipids.

The phospholipids can be selected from the group consisting of lecithins (phosphatidyl-choline), phosphatidyl-ethanolamines, phosphatidyl-serines and phosphatidyl-inositol and mixtures thereof, preferably from lecithins, phosphatidyl-ethanolamines and mixture thereof, even more preferably from lecithins.

The amphiphilic compound may also be an amphiphilic polymer selected from the group consisting of amphiphilic block polymers or graft polymer, preferably amphiphilic block polymers.

The amphiphilic block polymers can be a di-block amphiphilic polymer, preferably a non-ionic di-block amphiphilic polymer.

In particular, the amphiphilic block polymer can comprise a hydrophilic block and a hydrophobic block linked with each other.

The hydrophilic block may be selected from the group consisting of polyvinyl alcohol, polyalkylene glycol, polyvinyl pyrrolidone, polyacrylamide and derivatives thereof. Preferably, the hydrophilic block is selected from the group consisting of polyalkylene glycol.

The hydrophobic block may be selected from the group consisting of polyester, polyanhydride, polyamino acid, polyorthoester, polyphosphazine and derivatives thereof.

The amphiphilic compound may also be an amphiphilic surfactant, preferably a polymeric amphiphilic surfactant.

According to the present invention, the term "polymeric amphiphilic surfactant" means the surfactant comprises a part in its structure that is polymeric.

The amphiphilic compound may be a non-ionic amphiphilic surfactant preferably selected from the group consisting of sorbitol esters of C₈-C₂₄ fatty acids, esters of C₈-C₂₄ fatty acids and ethoxylated sorbitan containing from 4 to 40 ethylene oxide, oxyethylenated C₈-C₂₄ alcohol, esters of tocopherol and polyalkylene glycol and mixtures thereof.

Preferably, the amphiphilic compound is a non-ionic amphiphilic surfactant selected from esters of C₈-C₂₄ fatty acids and ethoxylated sorbitan containing from 4 to 40 ethylene oxide, more preferably from esters of C₁₂-C₁₈ fatty acids and ethoxylated sorbitan containing from 4 to 40 ethylene oxide, esters of tocopherol and polyalkylene glycol, preferably esters of tocopherol and polyethylene glycol, and mixtures thereof.

According to the present invention, the term tocopherol may include α-tocopherol, D-α-tocopherol, β-tocopherol, γ-tocopherol and δ-tocopherol, in particular D-α-tocopherol.

Preferably, esters of tocopherol and polyalkylene glycol are obtained from esterification of acid esters of tocopherol and polyalkylene glycol.

Acid esters of tocopherol can be chosen among the group consisting of tocopheryl acid succinates, tocopheryl acid citraconates, tocopheryl acid methyl citraconate, tocopheryl acid itaconate, tocopheryl acid maleate.

Preferably, esters of tocopherol and polyalkylene glycol are chosen among the group consisting of tocopherol polyethylene glycol succinate (TGPS), tocopherol sebacate polyethylene glycol, tocopherol citraconate polyethylene glycol, tocopherol methyl citraconate polyethylene glycol, tocopherol itaconate polyethylene glycol, tocopherol maleate polyethylene glycol and mixtures thereof.

Preferably, the non-ionic amphiphilic surfactant is chosen among tocopherol polyethylene glycol succinate (TGPS) and its derivatives, especially D-α-tocopherol polyethylene glycol succinate (TGPS), more specifically -α-tocopherol polyethylene glycol succinate having 1000 oxide ethylene units.

Preferably, the amphiphilic compound can be chosen from the group consisting of phospholipids, such as lecithins and phosphatidyl-ethanolamines, non-ionic amphiphilic block polymers, preferably non-ionic amphiphilic di-block polymers, non-ionic surfactant chosen from the group consisting of sorbitol esters of C₈-C₂₄ fatty acids, esters of C₈-C₂₄ fatty acids and ethoxylated sorbitan containing from 4 to 40 ethylene oxide, esters of tocopherol and polyalkylene glycol and mixtures thereof.

More preferably, the amphiphilic compound can be chosen from the group consisting of phospholipids, such as lecithins, non-ionic amphiphilic di-block polymers, esters of C₈-C₂₄, especially C₁₂-C₁₈, fatty acids and ethoxylated sorbitan containing from 4 to 40 ethylene oxide, esters of tocopherol and polyalkylene glycol, especially from the group consisting of phospholipids and, esters of tocopherol and polyalkylene glycol.

Even more preferably, the amphiphilic compound can be chosen from the group consisting of lecithins and esters of tocopherol and polyalkylene glycol.

The amphiphilic compound can be an amphiphilic lipid, preferably phospholipids, or a polymeric amphiphilic surfactant, preferably esters of tocopherol and polyalkylene glycol.

The amphiphilic compound can be present in the composition of the present invention at a concentration ranging from 5 to 90% by weight, preferably ranging from 10 to 80% by weight relative to the total weight of the composition.

According to the present invention, the composition further comprises one or more water-soluble solutes.

The water-soluble solutes are preferably chosen from physiologically acceptable water-soluble excipients, more preferably from pharmaceutical, dermatological and/or cosmetic physiologically acceptable water-soluble excipients.

According to the present invention, the feature "physiologically acceptable excipient" means an excipient that is compatible with the body, especially with the skin, the mucosae, especially oral mucosa, and/or the integumentary appendages.

Preferably, the water-soluble solutes are selected from pharmaceutical physiologically acceptable water-soluble excipients.

The water-soluble solutes can have a molecular weight ranging from 50 Da to 2.5 × 10⁶ g/mol, preferably ranging from 50 to 50,000 g/mol.

The water-soluble solutes are soluble in water at atmospheric pressure and at a temperature ranging from 20° to 37°C.

The physiologically acceptable water-soluble excipients are preferably water-soluble fillers.

The water-soluble solutes are preferably fillers selected from the group consisting of water-soluble salts, such as sodium chloride, sugar alcohols, such as mannitol, sorbitol, microcrystalline cellulose, lactose, polysaccharides such as sucrose, glucose, mannose, dextrans, starch saccharification products, water soluble polymers such as polyethylene glycol, polyvinylpyrrolidone and mixtures thereof.

Preferably, the water-soluble solutes are monosaccharides and polysaccharides such as sucrose, glucose, mannose, dextrans.

The composition preferably contains one or more amphiphilic compounds chosen among the group consisting of lipids, surfactants, in particular polymeric surfactants, and mixtures thereof, and one or more water-soluble solutes chosen among the group consisting of polysaccharides.

Preferably, the composition contains one or more amphiphilic compounds chosen among the group consisting of lipids, esters of tocopherol and polyalkylene glycol, and mixtures thereof, and one or more water-soluble solutes chosen among the group consisting of polysaccharides.

The water-soluble solutes can be present in the composition of the present invention at a concentration ranging from more than 0 to 70% by weight, preferably ranging from more than 0 to 60% by weight relative to the total weight of the composition.

Preferably, the composition is free of organic solvent.

According to the present invention, the feature "free of organic solvent" means that the composition does not comprise any organic solvent.

In others words, the aforementioned method preferably comprises cryomilling a composition free of organic solvent comprising:
- one or more amphiphilic compounds preferably selected from the group consisting of polymers, surfactants, lipids and mixtures thereof, preferably chosen from surfactants, in particular polymeric surfactants, and lipids, even more preferably selected from esters of tocopherol and polyalkylene glycol and lipids, especially tocopherol polyethylene glycol succinate and phospholipids,
- one or more water-soluble solutes preferably selected from the group consisting of physiologically acceptable water-soluble excipients, more preferably from pharmaceutical, dermatological and/or cosmetic physiologically acceptable water-soluble excipients, especially from pharmaceutical physiologically acceptable water-soluble excipients.

Preferably, the composition does not comprise an organic solvent chosen from the group consisting of alcohols like methanol, ethanol, propanol and so on, acetone, chloroform, ethers, tetrahydrofuran, dichloromethane.

In particular, the method for preparing the solid premix is a solvent free process, i.e. the method does not involve the implementation of any organic solvent at any of its steps.

The composition can further contain one or more lipids that are different from the lipids previously described.

In that respect, the composition can comprise one or more sterols, preferably cholesterol.

Preferably, the composition comprises:
a) one or more amphiphilic compounds selected from the groups consisting of polymers, surfactants, lipids and mixtures thereof, preferably from surfactants, in particular polymeric surfactants, and lipids, even more preferably selected from esters of tocopherol and polyalkylene glycol and lipids, especially tocopherol polyethylene glycol succinate and phospholipids,
b) one or more water-soluble solutes preferably selected from monosaccharides and polysaccharides such as sucrose, glucose, mannose, dextrans, more preferably glucose,
c) one or more lipids chosen in the group consisting of sterols, preferably cholesterol.

According to the aforementioned embodiment, the composition is preferably free of organic solvent.

The composition of the present invention can further comprise one or more active ingredients, especially selected from the group consisting of biological active ingredients, cosmetic active ingredients, dermatological active ingredients and mixtures thereof, more especially biological active ingredients and cosmetic ingredients, even more specially biological active ingredients.

According to the present invention, the term "biological active ingredient" means a compound that affects biological processes in a way that has an impact on body functions.

The biological active ingredient can be selected from the group consisting of pharmaceutical active ingredients, enzymes, proteins and mixtures thereof, preferably from pharmaceutical active ingredients.

According to the present invention, the term "pharmaceutical active ingredient" means any substance or its salts thereof used in a finished pharmaceutical product, intended to provide a pharmacological activity or to otherwise have direct effect in the diagnosis, cure, mitigation, treatment or prevention of disease, or to have direct effect in restoring, correcting or modifying physiological functions in human beings.

The pharmaceutical active ingredients may be hydrophilic or hydrophobic.

The pharmaceutical active ingredient can be chosen from the group consisting of any hydrophilic, lipophilic and amphiphilic pharmaceutically active ingredients either alone or in combination.

Preferably, the composition comprises one or more lipophilic pharmaceutical active ingredients.

The pharmaceutical active ingredients may be lipophilic and/or hydrophilic molecules and chosen among any category of drugs, preferably immunosuppressive agents, anticancer agents and antifungal agents.

The pharmaceutical active ingredients may be chosen among the group consisting of cyclosporine, voriconazole, natamycin, itraconazole and their pharmaceutically acceptable salts, especially cyclosporine, voriconazole and their pharmaceutically acceptable salts

### Method for preparing a nanosystem vehicle

As previously detailed, the present invention also relates to a method for preparing a nanosystem vehicle comprising:
i) cryomilling a composition comprising one or more amphiphilic compounds, as previously defined, and one or more water-soluble solutes, as previously defined, in order to obtain a solid premix,
ii) dispersing the solid premix into an aqueous medium.

Cryomilling is implemented in the same conditions as those previously detailed for the method for preparing the solid premix.

The dispersion of the solid premix into an aqueous medium can be performed at atmospheric pressure and at a temperature ranging from lower than 0°C to 90°C, more preferably 0°C to 80°C.

Preferably, the aqueous medium can be an aqueous solution or water.

The aqueous solution can comprise water at a concentration of 30 to 99% by weight, preferably at a concentration of 40 to 99% by weight, relative to the total weight of the aqueous solution.

The nanosystem vehicle prepared according to the aforementioned method can be a vesicular nanosystem.

The vesicular nanosystem is preferably selected from the group consisting of micelles, liposomes, niosomes, sphingosomes, transferosomes and ethosomes.

Preferably, the vesicular nanosystem can be selected from the group consisting of micelles, liposomes and niosomes, more preferably from micelles and liposomes, even more preferably from polymeric micelles and liposomes.

In case when multilamellar vesicles, especially in bilayer vesicles structures such as liposomes, are obtained after dispersing the solid premix into an aqueous medium, a conversion into unilamellar vesicles can be implemented, particularly by extrusion or ultrasonication.

Preferably, when multilamellar liposomes are obtained after dispersing the solid premix into an aqueous medium, a conversion into unilamellar liposomes can advantageously be implemented.

In other words, the aforementioned method can advantageously be a method for preparing unilamellar vesicles, in particular unilamellar liposomes, comprising:
i) cryomilling a composition comprising one or more amphiphilic compounds, as previously defined, and one or more water-soluble solutes, as previously defined, in order to obtain a solid premix,
ii) dispersing the solid premix into an aqueous medium in order to obtain multilamellar vesicles, in particular multilamellar liposomes,
iii) converting said multilamellar vesicles into unilamellar vesicles.

Converting said multilamellar vesicles into unilamellar vesicles can be performed by extrusion or not, preferably not by extrusion.

In particular, converting multilamellar vesicles into unilamellar vesicles can be performed by ultrasonication.

The nanosystem vehicles prepared according to the aforementioned method can also be lipid based nanoparticulate carriers such as solid lipid nanoparticles or nanostructured lipid carriers.

The lipid based nanoparticulate carriers obtained with the aforementioned method exhibit the advantage of being stable and uniform.

Preferably, the present invention is directed to a method for preparing nanosystem vehicles chosen from the group consisting of vesicular nanosystems or lipid based nanoparticles.

The method is advantageously a solvent free process.

In other words, the aforementioned method advantageously does not comprise the implementation of an organic solvent.

Preferably, the present invention is directed to a method for preparing a nanosystem vehicle, preferably chosen from the group consisting of micelles, liposomes and niosomes, more preferably from micelles and liposomes, even more preferably from liposomes, comprising:
i) cryomilling a composition comprising:
   a) one or more amphiphilic compounds selected from the groups consisting of polymers, surfactants, lipids, and mixtures thereof, more preferably polymers, such as di-block, tri-block or any other pharmaceutically and/or cosmetically acceptable amphiphilic polymers, surfactants, in particular polymeric surfactant, and lipids, even more preferably selected from esters of tocopherol and polyalkylene glycol and lipids, especially tocopherol polyethylene glycol succinate and phospholipids,
   b) one or more water-soluble solutes preferably selected from the group consisting of physiologically acceptable water-soluble excipients, more preferably from pharmaceutical, dermatological and/or cosmetic physiologically acceptable water-soluble excipients, especially from pharmaceutical physiologically acceptable water-soluble excipients;
      in order to obtain a solid premix,
ii) dispersing the solid premix into an aqueous medium.

One or more active ingredients as previously defined can be added in said composition and/or aqueous medium.

The composition can further comprise one or more active ingredients as previously described, especially one or more biological active ingredients, preferably selected from the group consisting of pharmaceutical active ingredients, enzymes, proteins and mixtures thereof, even more preferably pharmaceutical active ingredients.

Alternatively, the aforementioned method can further comprise adding one or more active ingredients, as previously described, especially one or more biological active ingredients such as pharmaceutical active ingredients, after cryomilling preferably in said aqueous medium.

The aqueous medium can comprise one or more active ingredients as previously described, especially one or more biological active ingredients, preferably selected from the group consisting of pharmaceutical active ingredients, enzymes, proteins and mixtures thereof, even more preferably pharmaceutical active ingredients as previously defined.

Preferably, one or more pharmaceutical active ingredients as previously defined may be present in said composition and/or in said aqueous medium.

Preferably, one or more hydrophilic pharmaceutical active ingredients may be present in said composition and/or in said aqueous medium.

Preferably, one or more lipophilic pharmaceutical active ingredients may be present in said composition.

### Premix

Another aspect of the present invention relies on a solid premix obtainable from a method comprising cryomilling a composition comprising one or more amphiphilic compounds, as previously disclosed, and one or more water-soluble solutes, as previously defined.

The solid premix is then ready to be dispersed into an aqueous medium, preferably water.

Preferably, the solid premix contains one or more active ingredients as previously described, especially one or more biological active ingredients, preferably selected from the group consisting of pharmaceutical active ingredients, enzymes, proteins and mixtures thereof, even more preferably from pharmaceutical active ingredients.

Preferably, the pharmaceutical active ingredient is lipophilic and may be chosen among any category of drugs, preferably among the group consisting of immunosuppressive agents, anti-inflammatory, anticancer agents and antifungal agents, preferably anticancer agents.

The average particle size of the solid premix prior to hydration ranges from 1 µm to 300 µm, preferably from 1 µm to 200 µm, even more preferably from 5 µm to 100 µm.

According to the present invention, the term "prior to hydration" means that the average particle size corresponds to the solid state premix powder before being dispersed into the aforementioned aqueous medium.

The average particle size may be measured as a weight average particle size with microscopy and sifting method, using sieves with known pore sizes.

X-ray diffraction analysis shows that the spectrum of the premix obtained from the method according to the present invention is different to the spectrum of each of the individual component of the premix.

DSC analysis shows that cryomilling implemented in the method of the present invention is able to obtain a uniform mixture of the components of the premix at submicron level.

The solid premix preferably exhibits a Carr's index ranging from 16 to 20.

The method for obtaining said solid premix preferentially displays the same features as those previously defined.

### Use of the solid premix

As previously detailed, the solid premix may be used in therapeutic application, preferably in order to deliver one or more biological active substances, preferably one or more pharmaceutical active ingredients, in the body, especially one or more pharmaceutical active ingredients to targeted sites in the body.

Therefore, another subject-matter of the present invention concerns the solid premix as previously described for its use in drug delivery, preferably in drug delivery for targeted sites in the body, containing one or more biological active ingredients chosen from pharmaceutical active ingredients as previously defined.

Especially, the invention concerns the solid premix as previously described, i.e. obtainable from the method previously defined, for its use in drug delivery in the body, preferably in drug delivery for targeted sites in the body, containing one or more biological active ingredients chosen from pharmaceutical active ingredients as previously defined.

The solid premix according to the present invention can also be used for non-therapeutic purposes as well.

In that respect, the invention also deals with the use of a solid premix as previously described for a non-therapeutic application, in particular for a dermatological or cosmetic application, especially a cosmetic application.

Preferably, the solid premix as previously described is used for a non-therapeutic application selected from the group consisting of:
- loading one or more biological active ingredients, preferably one or more pharmaceutical active ingredients,
- stabilizing one or more biological active ingredients, especially proteins or enzymes, such as messenger RNA (mRNA), or
- preparing a topical non-therapeutic composition, preferably a dermatological or cosmetic composition, more preferably a cosmetic composition.

Preferably, the solid premix is used for loading one or more biological active ingredients, preferably one or more pharmaceutical active ingredients.

Preferably, the solid premix is used for preparing a topical non-therapeutic composition, such as a dermatological or cosmetic composition, more preferably a cosmetic composition.

In other words, another subject-matter of the present invention is directed to a method for preparing a topical non-therapeutic composition, such as a dermatological or a cosmetic composition, preferably a cosmetic composition, comprising:
- cryomilling a composition comprising one or more amphiphilic compounds, as previously defined, and one or more water-soluble solutes, as previously defined, in order to obtain a solid premix,
- dispersing the solid premix into an aqueous medium in order to obtain a nanosystem vehicle as previously defined,
- adding one or more dermatologic active ingredients or cosmetic active ingredients in said composition or aqueous medium.

### Nanosystem vehicles

The invention is also directed to a nanosystem vehicle, obtainable from the method previously defined, for its use in a therapeutic treatment and/or *in vivo* diagnosis method, especially therapeutic treatment, comprising one or more biological active ingredients as previously disclosed, preferably chosen in the group consisting of pharmaceutical active ingredients.

Preferably, the invention relates to a liposome vehicle, for its use in a therapeutic treatment and/or *in vivo* diagnosis method, especially therapeutic treatment, comprising one or more biological active ingredients as previously disclosed, preferably chosen in the group consisting of pharmaceutical active ingredients.

According to this embodiment, the amphiphilic compound is preferably selected among the group consisting of lipids.

Preferably, the invention relates to a micelle vehicle, for its use in a therapeutic treatment and/or *in vivo* diagnosis method, especially therapeutic treatment, comprising one or more biological active ingredients as previously disclosed, preferably chosen in the group consisting of pharmaceutical active ingredients as preferably defined.

According to this embodiment, the amphiphilic compound is preferably selected among the group consisting of surfactants.

Preferably, the pharmaceutical active ingredient is lipophilic and/or hydrophilic molecule may be chosen among any category of drugs, more preferably immunosuppressive agents, anti-inflammatory, anticancer agents and antifungal agents, preferably anticancer agents.

Another subject of the present invention pertains to the use of a nanosystem vehicle, obtainable from the method previously defined, in order to impart cosmetic properties to human keratin materials, preferably to skin, comprising one or more cosmetic active ingredients.

The nanosystem vehicle can have a contact angle on a solid substrate, which is lower than 25°, preferably lower than 22°.

The contact angle of the nanosystem vehicle can be assessed by sessile drop technique using Lorentzen and Wettre, type 28 optical tensiometer.

The following examples are given as illustrations of the present invention.

### Example 1: Micelles and liposomal formulation of lipophilic drugs

### A. TPGS (D-α-Tocopherol polyethylene glycol 1000 succinate) based polymeric micelles of lipophilic drugs

Non-ionic polymeric surfactant TPGS and the tested lipophilic drug were cryomilled along with glucose (1 : 0.6 parts of TPGS) as a water soluble solute using SamplePrep^{®} freezer mill 6770. The obtained premix was added to aqueous phase to form micelles. The characterization parameters of the polymeric micelles are given in Table 1.

**Table 1: Micelle formulations**

| **Drug** | **Log P** | **TPGS concentration** | **Drug targeted concentration** | **Drug encapsulated concentration** | **Entrapment efficiency %** | **Particle size (Zav, nm)** |
|---|---|---|---|---|---|---|
| Cyclosporine | 4.12 | 30 mg/ml | 3 mg/ml | 2.99 mg/ml | 99.90 ± 3.2 | 11.61 ± 1.7 |
| Voriconazole | 1.75 | 20 mg/ml | 3 mg/ml | 2.49 mg/ml | 82.66 ± 6.8 | 11.57 ± 1.2 |
| N atamycin | - | 5 mg/ml | 0.2 mg/ml | 0.10 mg/ml | 52.36 ± 0.1 | 11.89 ± 4.5 |

The Z average of the particle size corresponds to the intensity weighted mean hydrodynamic size of the ensemble collection of particles measured by dynamic light scattering (DLS).

The drug content and entrapment efficiency have been determined by using Acquity^{®} Core UPLC^{®} system equipped with Xevo^{®} TQ-MS tandem quadrupole detector system.

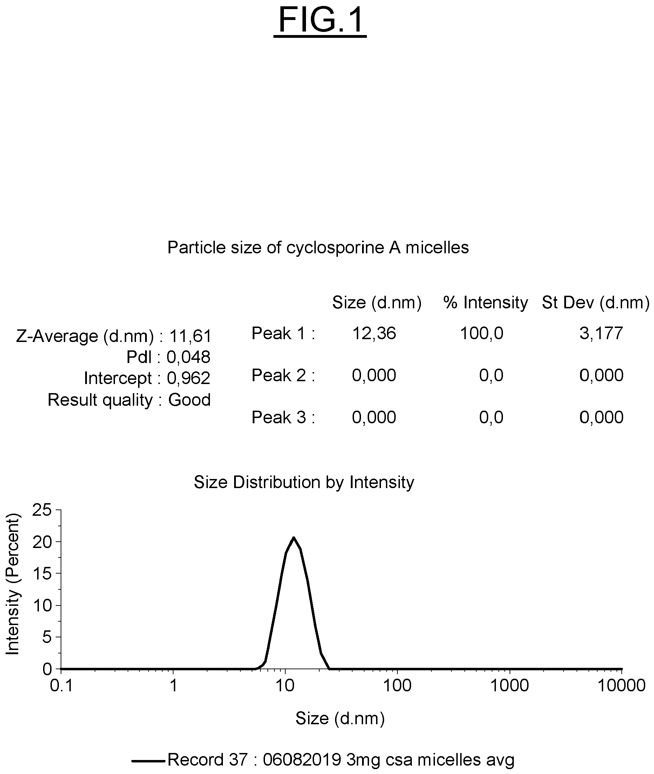

The intensity as a function of the size distribution of the cyclosporine micelle formulation has been plotted on Figure 1.

### B. Lipoid S-100 based lipophilic drugs liposomes

Lipoid S-100 (soyabean phophatidylcholine) and the tested lipophilic drug were cryomilled along with glucose as a water-soluble solute using SamplePrep^{®} freezer mill 6770. The obtained premix was added to aqueous phase to form multilamellar liposomes, which were further extruded to obtain small unilamellar vesicles. Probe ultra-sonication can also be used for this purpose. Characterization parameters were studied as described previously. The characterization parameters are shown in Table 2.

**Table 2: Liposomes formulations**

| **Drug** | **Lipoid S-100 concentration** | **Drug targeted concentration** | **Drug encapsulated concentration** | **Entrapment efficiency %** | **Particle size (Zav, nm)** |
|---|---|---|---|---|---|
| Cyclosporine | 60 mg/ml | 3 mg/ml | 2.99 mg/ml | 99.90 ± 4.8 | 86.83 ± 8.3 |
| Voriconazole | 50 mg/ml | 3 mg/ml | 3 mg/ml | 99.80 ± 2.4 | 75.47 ± 6.3 |

The drug content and entrapment efficiency have been determined by using Acquity Core UPLC^{®} system equipped with Xevo^{®} TQ-MS tandem quadrupole detector system. Malvern Zetasizer was used to determine particle size of the liposome.

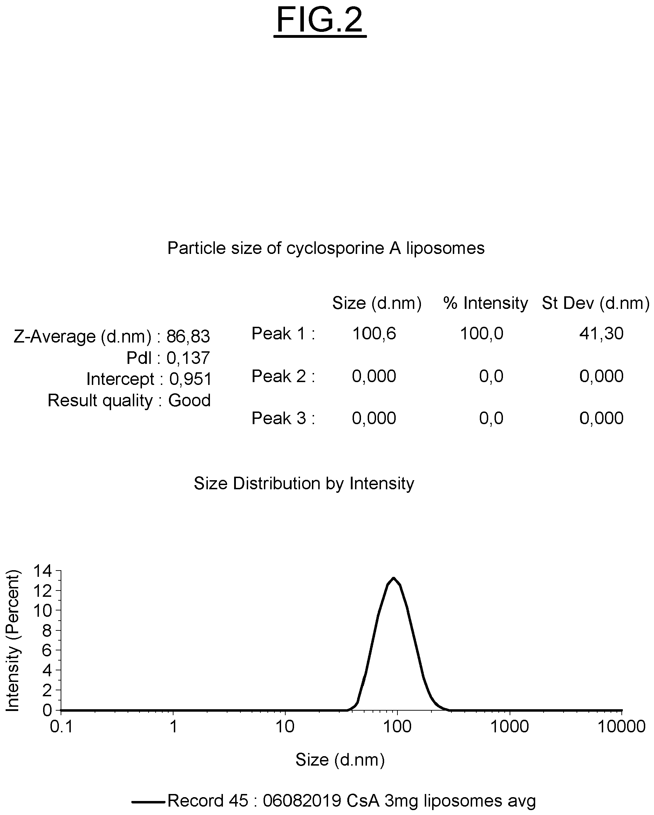

The intensity as a function of the size distribution of the cyclosporine liposome formulation has been plotted on Figure 2.

### Example 2: Contact angle measurement

### A. Micelles and liposomes of voriconazole

Micelles and liposomes of voriconazole have been prepared according to the method disclosed in example 1 and display the formulations characteristics described in Tables 1 and 2.

The contact angles of voriconazole alone, glucose, voriconazole micelle formulation and voriconazole liposome formulation on a solid surface were measured by sessile drop technique using Lorentzen and Wettre, type 28 optical tensiometer.

The purpose of measuring the contact angle is to determine the wetting properties of the formulation. Higher contact angles correspond to poor wetting whereas lower contact angle correspond to good wetting properties.

The results are given in Table 3.

**Table 3: Contact angles measurement**

| | **Contact angle** |
|---|---|
| Glucose | 0° |
| V oriconazole | 68.2° |
| Voriconazole micelle premix | 20.56° |
| Voriconazole liposome premix | 7.13° |

According to Table 3, glucose shows excellent wetting properties as its contact angle corresponds to 0°.

Voriconazole had a significantly higher contact angle than voriconazole micelle formulation and voriconazole liposome formulation, indicating enhanced wetting properties of both nanosystem formulations.

### B. Micelles and liposomes of itraconazole

Micelles and liposomes of itraconazole have been prepared according to the method described in example 1. The contact angles of itraconazole alone, glucose, itraconazole micelle formulation and itraconazole liposome formulation on a solid surface were measured by sessile drop technique using Lorentzen and Wettre, type 28 optical tensiometer.

The results are given in Table 4 below

**Table 4: Contact angles measurement**

| | **Contact angle** |
|---|---|
| Glucose | 0° |
| Itraconazole | 73.3 |
| Itraconazole micelle premix | 21.8° |
| Itraconazole liposome premix | 18.43° |

According to Table 4, glucose shows excellent wetting properties as its contact angle corresponds to 0°.

Itraconazole had a significantly higher contact angle than itraconazole micelle formulation and itraconazole liposome formulation, indicating enhanced wetting properties of both nanosystem formulations.

### Example 3: X-ray diffraction analysis (XRD) of lipophilic drugs premix

The premix obtained after cryomilling and individual ingredients were subjected to X-ray diffraction to identify the crystalline and amorphous components of the system and how the degree of crystallinity got affected by the method of the present invention.

All samples were measured on Empyrean (PANAlytical) diffractometer in capillary mode, with a focusing X-ray mirror for Cu_{Kα} radiation and a PIXcel3D area detector at ambient temperature.

### A. X-ray diffraction analysis of voriconazole micelle premix and the voriconazole liposome premix

### 1. X-ray diffraction analysis of voriconazole micelle premix

Voriconazole micelle premix was prepared according to the method disclosed in example 1 and was then subjected to X-ray diffraction. The voriconazole micelle premix displays the formulations characteristics described in Table 1.

Each of the individual components of the voriconazole micelle premix (voriconazole, glucose and TGPS alone) were also subjected to X-ray diffraction.

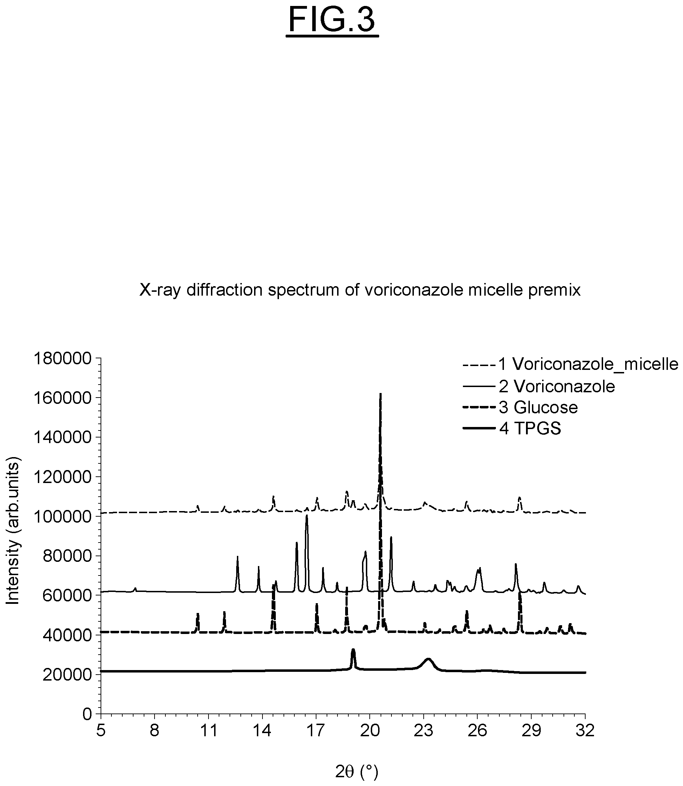

XRD of voriconazole micelle premix and its individual components were plotted on Figure 3 in order to ease the comparison.

Figure 3 shows that voriconazole and glucose (individually considered) have crystalline structure whereas TGPS alone was found to be almost amorphous but had two characteristic peaks (semi-crystalline).

In the premix, the intensity of signals of voriconazole was greatly reduced due to uniform mixing with amorphous TGPS thanks to the implementation of cryomilling.

### 2. X-ray diffraction analysis of voriconazole liposome premix

Voriconazole liposome premix was also prepared according to the method described in example 1 and was subjected to X-ray diffraction. The voriconazole liposome premix displays the formulations characteristics described in Table 2.

Each of the individual components of the voriconazole liposome premix (voriconazole, glucose and lipid alone) was also subjected to X-ray diffraction.

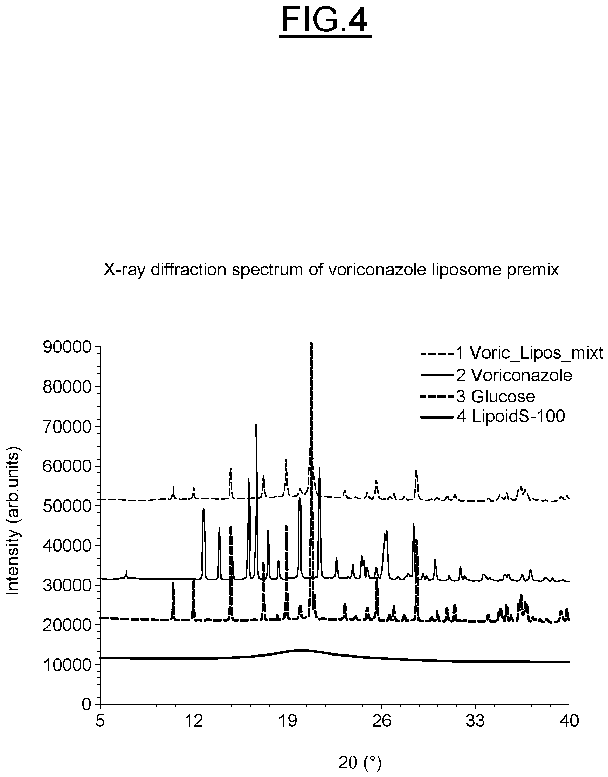

XRD of voriconazole liposome premix and its individual components were plotted on Figure 4 in order to ease the comparison.

According to Figure 4, voriconazole and glucose were found to have a crystalline structure whereas lipids alone were found to be amorphous.

In the premix, the intensity of signals of voriconazole was greatly reduced due to uniform mixing with amorphous lipids thanks to the implementation of cryomilling.

### B. X-ray diffraction analysis of cyclosporine micelle premix and cyclosporine liposome premix

### 1. X-ray diffraction analysis of cyclosporine micelle premix

Cyclosporine micelle premix was prepared according to the method described in example 1 and was subjected to X-ray diffraction. The cyclosporine micelle premix displays the formulations characteristics described in Table 1.

Each of the individual components of the cyclosporine micelle premix (cyclosporine, glucose and TGPS alone) was also subjected to X-ray diffraction.

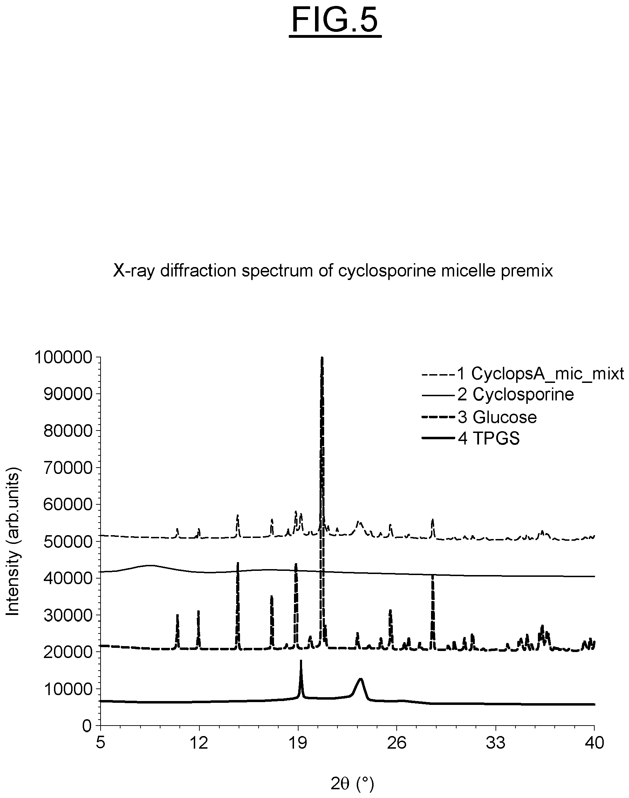

XRD of cyclosporine micelle premix and its individual components were plotted on Figure 5 in order to ease the comparison between premix and individual components.

According to Figure 5, cyclosporine was found to be amorphous, TGPS alone was found to be almost amorphous but had two characteristic peaks (semi-crystalline), glucose was found to be crystalline.

The cyclosporine micelle premix displays a different XRD to its individual components, especially cyclosporine alone.

### 2. X-ray diffraction analysis of cyclosporine liposome premix

Cyclosporine liposome premix was prepared according to the method described in example 1 and was subjected to X-ray diffraction. The cyclosporine liposome premix displays the formulations characteristics described in Table 2.

Each of the individual components of the cyclosporine liposome premix (cyclosporine, glucose and TGPS alone) was also subjected to X-ray diffraction.

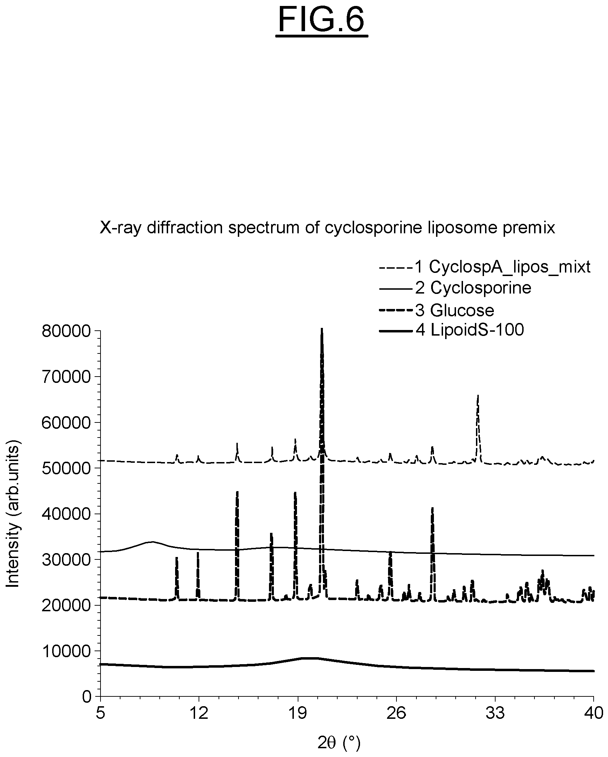

XRD of cyclosporine liposome premix and its individual components have been plotted on Figure 6 in order to ease the comparison.

According to Figure 6, cyclosporine and lipids were found to be amorphous and glucose was found to be crystalline.

The cyclosporine liposome premix displays a different XRD spectrum to its individual components, especially cyclosporine alone.

### Example 4: Differential scanning calorimetry (DSC)

The premixes obtained after cryomilling, their corresponding individual ingredients and the physical mixture of the active ingredient and the amphiphilic compounds were subjected to thermal analysis with a METTLER Toledo DSC3 STAR system.

### A. DSC analysis of voriconazole micelle premix, individual components and physical mixture

### 1. Tested specimens

Voriconazole micelle premix was prepared according to the method disclosed in example 1 and exhibits the formulation characteristics given in Table 1.

Voriconazole, glucose and TGPS were individually thermally analysed.

The physical mixture of TGPS, voriconazole and glucose was prepared by mixing the aforementioned components in solid state in a ratio similar to that in the premix.

### 2. Results

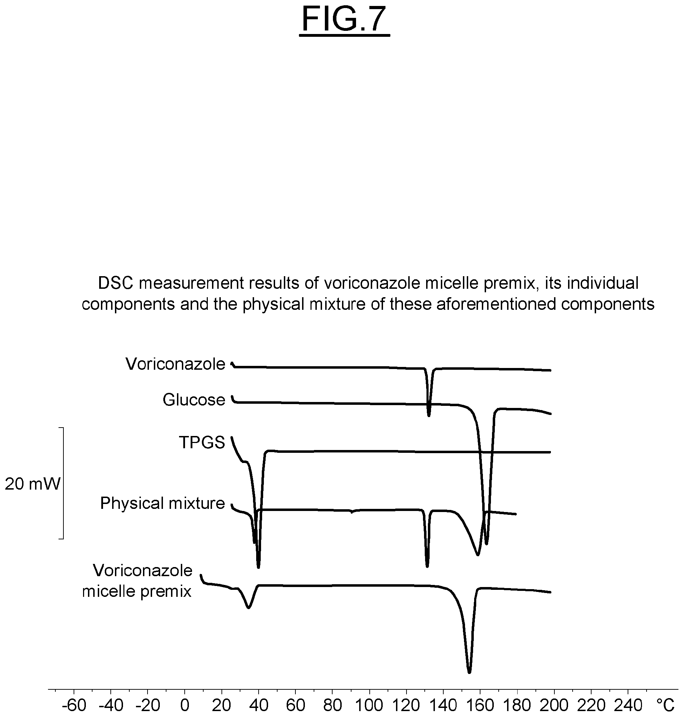

Figure 7 shows the DSC measurement results for voriconazole micelle premix, its individual components (glucose, TGPS and voriconazole) and the physical mixture of voriconazole, TGPS and glucose.

According to Figure 7, the DSC measurement of the physical mixture exhibits three endothermic peaks temperature, which correspond to the endothermic peaks of TGPS, voriconazole and glucose assessed alone.

The endotherm of voriconazole corresponding to its melting point is not observed in the premix. Indeed, the DSC measurements unveil that the premix exhibits two endothermic peaks temperature corresponding to TGPS and glucose at a lower intensity.

It bespeaks that a uniform mixture of TGPS, voriconazole and glucose at submicron level have been achieved due to cryomilling.

The DSC measurements also reveal that a shift of the endothermic peaks of TGPS and glucose to lower temperature region occurred in the premix. This shift ensues from the particle size reduction achieved with cryomilling.

### B. DSC analysis of voriconazole liposome premix, individual components and physical mixture

### 1. Tested specimens

Voriconazole liposome premix was prepared according to the method disclosed in example 1 and exhibits the formulation characteristics given in Table 2.

Voriconazole, glucose and soyabean phophatidylcholine (Lipoid S100) taken individually have also been thermally analysed

The physical mixture of lipids and voriconazole was prepared by mixing the aforementioned components in solid state in a ratio similar to that in the premix.

### 2. Results

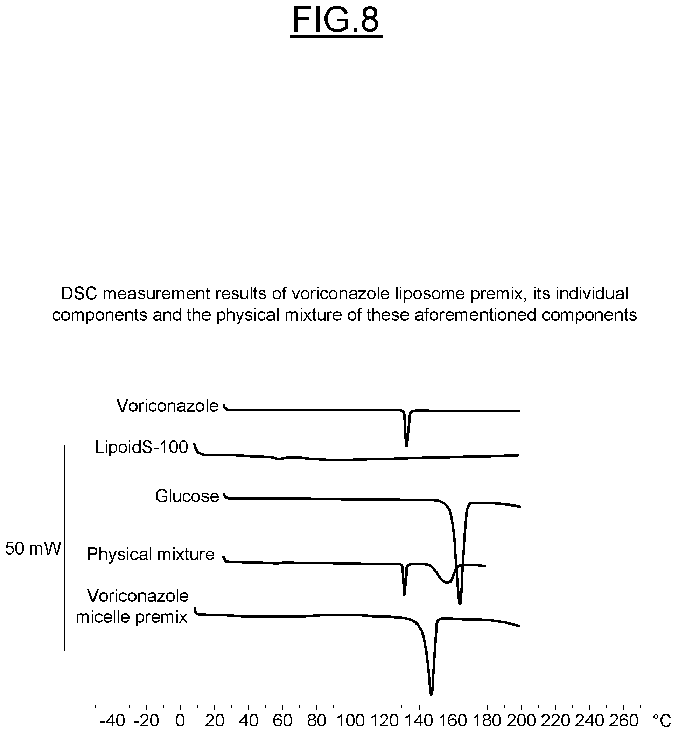

Figure 8 shows the DSC measurement results for voriconazole premix liposome, its individual components (glucose, lipoid-S100 and voriconazole) and the physical mixture of voriconazole, soyabean phophatidylcholine (Lipoid S100) and glucose.

According to Figure 8, the DSC measurement of the physical mixture exhibits two endothermic peaks temperature which correspond to the endothermic peaks of voriconazole and glucose assessed alone.

The endotherm of voriconazole corresponding to its melting point is not observed in the premix. Indeed, the DSC measurements unveil that the premix exhibits only one endothermic peak temperature corresponding to glucose at a lower intensity.

It bespeaks that a uniform mixture of lipid, voriconazole and glucose at submicron level have been obtained thanks to cryomilling.

The DSC measurements also reveal that a shift of the endothermic peak of glucose to lower temperature region occurred in the premix. This shift ensues from the particle size reduction achieved with cryomilling.

Hence, the DSC analysis shows that the voriconazole liposome premix does not display the same endothermic peaks temperature as the solid solution.

### C. DSC analysis of cyclosporine premix, individual components and physical mixture

### 1. Tested specimens

Cyclosporine micelle premix was prepared according to the method disclosed in example 1 and exhibits the formulations characteristics described in Table 1.

Cyclosporine, glucose and TGPS taken individually have also been thermally analysed.

The physical mixture of TGPS, cyclosporine and glucose was prepared by mixing the aforementioned components in solid state in a ratio similar to that in the premix

### 2. Results

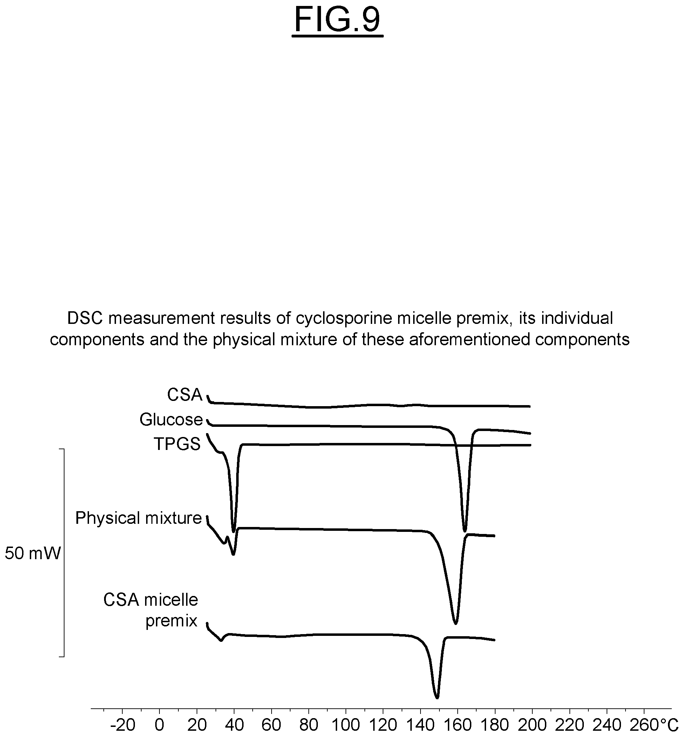

Figure 9 shows the DSC measurement results for cyclosporine premix micelle, its individual components (glucose, TGPS and cyclosporine) and the physical mixture of cyclosporine, TGPS and glucose.

The DSC analysis of cyclosporine (denoted CSA on Figure 9) did not reveal any noticeable endotherms due to its amorphous nature.

The DSC analysis of the premix exhibits the endothermic peaks temperature of TGPS and glucose at a lower intensity and in lower temperature region.

The shift of the aforementioned endothermic peaks to lower temperature in the premix ensues from the particle size reduction achieved with cryomilling.

The DSC analysis shows that the cyclosporine micelle premix does not display the same endothermic peaks temperature as the physical mixture.

## Claims

1. Method for preparing a solid premix comprising cryomilling a composition comprising one or more amphiphilic compounds and one or more water-soluble solutes.

2. Method according to Claim 1, **characterized in that** cryomilling is performed in a cryogenic fluid cooled environment.

3. Method according to Claim 1 or 2, **characterized in that** the amphiphilic compound is selected from the group consisting of polymers, surfactants, lipids and mixtures thereof, preferably from lipids, polymeric surfactants and mixtures thereof, even more preferably from lipids.

4. Method according to any of preceding claims, **characterized in that** said amphiphilic compound is an amphiphilic lipid selected from the group consisting of phospholipids, synthetic phospholipids, glycerolipids, sphingolipids, glycosphingolipids and mixtures thereof, preferably phospholipids.

5. Method according to any of preceding claims, **characterized in that** the amphiphilic compound is selected from the group consisting of phospholipids, amphiphilic block and/or graft copolymers, preferably non-ionic amphiphilic di-block polymers, non-ionic surfactant chosen from the group consisting of sorbitol esters of C₈-C₂₄ fatty acids, esters of C₈-C₂₄ fatty acids and ethoxylated sorbitan containing from 4 to 40 ethylene oxide, esters of tocopherol and polyalkylene glycol and mixtures thereof.

6. Method according to any of preceding claims, **characterized in that** the water-soluble solute is chosen from the group consisting of physiologically acceptable water-soluble excipients, preferably fillers selected from the group consisting of water-soluble salts, such as sodium chloride, sugar alcohols, such as mannitol, sorbitol, microcrystalline cellulose, lactose, polysaccharides, water soluble polymers and mixtures thereof.

7. Method according to any of preceding claims, **characterized in that** the composition is free of organic solvent.

8. Method for preparing a nanosystem vehicle comprising:
i) cryomilling a composition comprising one or more amphiphilic compounds, as defined according to Claims 1 or 3 to 5, and one or more water-soluble solutes, as defined according to Claim 1 or 6, in order to obtain a solid premix,
ii) dispersing the solid premix into an aqueous medium.

9. Method according to Claim 8, **characterized in that** said nanosystem vehicle is a vesicular nanosystem selected from the group consisting of micelles, liposomes, niosomes, sphingosomes, transferosomes and ethosomes, preferably from micelles and liposomes.

10. Method according to Claim 8 or 9, **characterized in that** when multilamellar vesicles are obtained after dispersing the solid premix into an aqueous medium, a conversion into unilamellar vesicles is implemented by extrusion or ultrasonication.

11. Method according to Claim 8, **characterized in that** said nanosystem vehicle is a lipid based nanoparticulate carriers preferably solid lipid nanoparticles or nanostructured lipid carriers.

12. Method according to Claims 8 to 11, **characterized in that** said composition further comprises one or more active ingredients, preferably selected from the group consisting of biological active ingredients, cosmetic active ingredients, dermatological active ingredients and mixtures thereof, more especially biological active ingredients.

13. Method according to Claims 8 to 11, **characterized in that** it further comprises adding one or more active ingredients, preferably one or more biological active ingredients such as pharmaceutical active ingredients, after cryomilling.

14. Solid premix obtainable from the method as defined according to any of Claims 1 to 7.

15. Solid premix according to Claim 14 for its use in drug delivery comprising one or more biological active ingredients chosen from pharmaceutical active ingredients.

16. Use of a solid premix according to Claim 14 for a non-therapeutic application selected from the group consisting of:
- loading one or more biological active ingredients, preferably one or more pharmaceutical active ingredients,
- stabilizing one or more biological active ingredients, or
- preparing a topical non-therapeutic composition, preferably a dermatological or cosmetic composition.

17. Nanosystem vehicle obtainable from the method as defined according to any of Claims 8 to 13, for its use in a therapeutic treatment and/or *in vivo* diagnosis method, comprising one or more biological active ingredients chosen in the group consisting of pharmaceutical active ingredients.

18. Use of a nanosystem vehicle, obtainable from the method as defined according to any of Claims 8 to 13 in order to impart cosmetic properties to human keratin materials, preferably to skin, comprising one or more cosmetic active ingredients.
